# EUROPEAN PATENT SPECIFICATION

(11) **EP 4 520 436 B1**
(45) Date of publication and mention of the grant of the patent: **09.09.2026**
(21) Application number: 23807653.3
(22) Date of filing: 16.05.2023
(51) Int. Cl.: B02C 18/14, A61F 13/15, B02C 18/16, D04H 1/04, D21B 1/06, B02C 18/18

(54) **PULVERIZATION DEVICE**
PULVERISIERVORRICHTUNG
DISPOSITIF DE PULVÉRISATION

(30) Priority: 17.05.2022 JP 2022081170
(43) Date of publication of application: 12.03.2025
(73) Proprietor: Zuiko Corporation, Ibaraki-shi, Osaka 5670082 (JP)
(72) Inventor: SHIMADA, Takahiro, Ibaraki-shi, Osaka 567-0082 (JP)
(74) Representative: Hasegawa, Kan
(86) International application number: PCT/JP2023/018344
(87) International publication number: WO 2023/224052

(56) References cited:
- WO-A1-2014/087902
- WO-A1-2015/037466
- JP-A- 2002 349 588
- JP-A- 2008 036 526
- JP-A- 2010 196 727
- JP-A- 2011 047 087
- JP-A- 2015 062 463
- JP-A- 2015 062 463
- JP-A- 2017 109 184
- JP-A- 2020 076 427
- US-B2- 10 098 791
- US-B2- 10 814 333

## Description

### TECHNICAL FIELD

The present invention relates to a pulverization device that pulverizes a pulp sheet serving as a raw material for an absorbent body and produces pulp fiber.

### BACKGROUND ART

Pulverization devices are known that cause rotary blades to rotate and pulverize a pulp sheet using the rotary blades, thereby producing pulp fiber for manufacturing an absorbent body, as with the pulverization device disclosed in JP 2015 062463 A.

JP 2017 109184 A discloses a defibrating machine that crushes a raw material sheet into a fibrous material, comprising: a unraveling roll having a plurality of cutter plates and being rotated and driven in one direction; and a raw material sheet supply mechanism for introducing a raw material sheet toward an outer circumference of the unraveling roll, the cutter plate being provided with a plurality of rotary blades arranged in a circumferential direction of an outer peripheral portion and a plate portion supporting the rotary blade. The rotary blade 13a is formed such that the length of the defibrating roll in the axial direction X is longer than the plate portion, and between the plate portions of the adjacent cutter plate, the circumference of the defibrating roll is provided. A continuous space is formed over the entire circumference in the direction Y.

### SUMMARY OF THE INVENTION

### <Technical Problem>

As a typical problem to be addressed in such pulverization devices, there is demand to realize high processing capacity in a compact device.

However, in doing so, it is possible for adverse events to occur, such as the pulp fiber burning or the pulverization of the pulp sheet being inadequate, depending on operating conditions. In order to avoid the occurrence of such adverse events, pulverization devices are actually designed to have considerable tolerances. Therefore, there is room for improvement in enhancing performance of pulverization devices.

It is an object of the present invention to provide a pulverization device that pulverizes a pulp sheet serving as a raw material for an absorbent body and produces pulp fiber, the pulverization device being compact, having high processing capacity, and making it possible to suppress adverse events such as the pulp fiber burning or pulverization of the pulp sheet being inadequate.

### <Solution to Problem>

A pulverization device according to the present invention pulverizes a pulp sheet and produces pulp fiber. The pulverization device comprises a rotary shaft, a plurality of rotary blades, a motor, and a casing. The plurality of rotary blades are attached to the rotary shaft. The rotary blades is configured to rotate together with the rotary shaft and pulverize the pulp sheet. The motor is configured to cause the rotary shaft to rotate. The casing has a supply port through which the pulp sheet is configured to be supplied and a discharge port through which the pulp fiber is configured to be discharged. The casing accommodates the rotary blades attached to the rotary shaft. A pulverization space index I [m³/kg] is defined using numerical expression (V [m³] × N [rpm] × 60 [min]) ÷ Amax [kg/h], where V [m³] is a volume of a void space within a virtual cylinder, N [rpm] is a rate of rotation of the rotary shaft, and Amax [kg/h] is a maximum amount of material pulverized by the pulverization device. The virtual cylinder is an imaginary cylinder for which a radius is a distance from a center of the rotary shaft to an end section of the rotary blade disposed furthest away from the center of the rotary shaft in a radial direction of the rotary shaft and for which a height is a segment distance within which the plurality of rotary blades are disposed in an axial direction of the rotary shaft. The volume V [m³], the rate of rotation N [rpm], and the maximum pulverization amount Amax [kg/h] in the definition above are set so that the pulverization space index I [m³/kg] is equal to or greater than 12.0.

### <Advantageous Effects of Invention>

The inventors discovered that having the pulverization space index I be equal to or greater than 12.0 m³/kg makes it possible to suppress the occurrence of adverse events such as the pulp fiber burning and pulverization of the pulp sheet being markedly inadequate during pulverization. Because the volume V [m³], the rate of rotation N [rpm], and the maximum pulverization amount Amax [kg/h] are set so that the aforementioned condition is satisfied in the pulverization device according to the first aspect, it is possible to suppress the occurrence of adverse events such as the pulp fiber burning and pulverization of the pulp sheet being markedly inadequate even without excessive safety tolerances.

### BRIEF DESCRIPTION OF THE DRAWINGS

[Fig. 1] A schematic diagram of an absorbent body manufacturing apparatus having a pulverization device according to one embodiment of the present invention.
[Fig. 2] A schematic diagram in which the pulverization device according to one embodiment of the present invention is viewed along an axial direction of a rotary shaft of the pulverization device.
[Fig. 3] A schematic top view of a rotary body and bearing devices of the pulverization device shown in Fig. 2, where a casing and bearing boxes are illustrated using dashed and double-dotted lines.
[Fig. 4] A schematic diagram in which rotary blades (rotary blade plates) that are attached to the rotary shaft of the pulverization device shown in Fig. 2 are viewed along the axial direction of the rotary shaft.
[Fig. 5] A diagram in which arcuate surfaces, where blade tips of the rotary blades of the rotary body of the pulverization device shown in Fig. 2 are disposed, are illustrated in a planar manner, the diagram being for illustrating a state in which the blade tips of the rotary blades are disposed.
[Fig. 6] A diagram in which a region including a quadrangle indicated by reference symbol VI in Fig. 3 is partially enlarged, the diagram being for illustrating a void space.
[Fig. 7] A diagram in which a maximum pulverization amount and a pulverization space index from when a pulp sheet is pulverized using test equipment for the pulverization device (i.e., data shown in table 1) are plotted on a graph in which the maximum pulverization amount is taken on a horizontal axis and the pulverization space index is taken on a vertical axis, where symbols used for each plot indicate quality of resulting pulp fiber.
[Fig. 8] A schematic diagram in which the rotary blades (rotary blade plates) used in testing for obtaining the graph shown in Fig. 7 (i.e., data shown in table 1) are viewed along the axial direction of the rotary shaft.
[Fig. 9] A schematic diagram in which a pulverization device according to modification A is viewed along an axial direction of a rotary shaft of the pulverization device.

### DESCRIPTION OF EMBODIMENTS

Embodiments of a pulverization device according to the present invention shall be described below with reference to the accompanying diagrams.

The embodiments described below are merely examples of the present invention and do not limit the scope of the present invention.

### (1) Absorbent body manufacturing apparatus

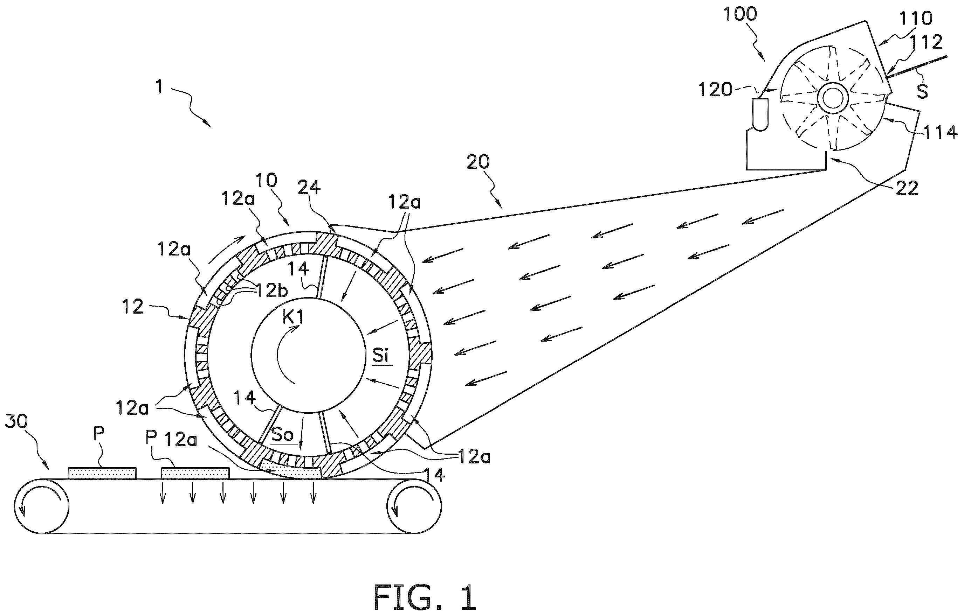

An absorbent body manufacturing apparatus 1 having a pulverization device 100 according to one embodiment of the present invention shall be described with reference to Fig. 1. Fig. 1 is a schematic diagram of the absorbent body manufacturing apparatus 1.

An absorbent body P manufactured by the absorbent body manufacturing apparatus 1 is an article obtained by molding pulp fiber into a prescribed shape. The absorbent body P is an article that absorbs urine, blood, or another body fluid, the article being used in, e.g., a disposable diaper or a sanitary napkin.

The absorbent body manufacturing apparatus 1 includes the pulverization device 100, a fiber integration device 10, a duct 20, and a suction conveyor 30 (see Fig. 1). The pulverization device 100 pulverizes a pulp sheet S obtained by agglutinating pulp fiber and forming the resulting article into a sheet, and produces pulp fiber. Specifically, the pulverization device 100 is a device that unravels (breaks down) the sheet-formed pulp fiber into flocs. The duct 20 functions as a pulp fiber passage through which the pulp fiber produced by the pulverization device 100 is guided from the pulverization device 100 to the fiber integration device 10. The fiber integration device 10 molds the pulp fiber pulverized by the pulverization device 100 into absorbent bodies P of prescribed shape. The absorbent bodies P manufactured by the fiber integration device 10 are transported by the suction conveyor 30 to a step in the latter section of the absorbent body manufacturing apparatus 1.

The pulverization device 100 shall be described in detail further below.

The duct 20 has a first opening 22 disposed furthest upstream on a pulp fiber transport path, and a second opening 24 disposed furthest downstream on the pulp fiber transport path (see Fig. 1). The first opening 22 is connected to a pulp fiber discharge port 114 formed in a casing 110 (described further below) of the pulverization device 100. The second opening 24 is disposed so as to face a portion of an outer peripheral surface of a fiber integration drum 12 (described further below) of the fiber integration device 10. The pulp fiber flowing into the duct 20 through the first opening 22 is supplied through the second opening 24 into recesses 12a (described further below) provided to the outer peripheral surface of the fiber integration drum 12.

The fiber integration device 10 is mainly provided with the fiber integration drum 12 (see Fig. 1).

The fiber integration drum 12 is a cylindrical rotary drum having a hollow interior. An outer peripheral portion of the fiber integration drum 12 rotates in the direction of arrow K1 (see Fig. 1) about a rotary shaft extending along a center of the cylindrical fiber integration drum 12. The pulp fiber is aggregated on the outer peripheral surface of the fiber integration drum 12, and the recesses 12a, which are adapted for molding the pulp fiber into absorbent bodies P of the prescribed shape, are formed in the outer peripheral surface of the fiber integration drum 12. The recesses 12a have a shape corresponding to the absorbent bodies P so that the shape of the pulp fiber retrieved from the recesses 12a will assume the prescribed shape for the absorbent bodies P. The exterior and interior of the fiber integration drum 12 communicate via holes 12b formed in bottom sections (radially inner sides) of the recesses 12a (see Fig. 1). The holes 12b are designed to have dimensions that allow air in pulp-fiber-containing air, which flows into the recesses 12a from the duct 20, to pass through the holes 12b without allowing the pulp fiber to pass therethrough.

A plurality of spaces divided by partition walls 14 disposed inside the fiber integration drum 12 are formed inside the fiber integration drum 12 along a circumferential direction of the fiber integration drum 12 (see Fig. 1). An intake space Si (see Fig. 1), which is one of the plurality of spaces, is a (negative-pressure) space having a lower pressure than the exterior of the fiber integration drum 12, internal air in the intake space Si being suctioned through an opening (not shown). Air is taken into the intake space Si from a space outside of the fiber integration drum 12 via the holes 12b formed in the bottom sections of the recesses 12a. A blowout space So (see Fig. 1), which is one of the plurality of spaces, is disposed adjacent to the intake space Si in the circumferential direction of the fiber integration drum 12 and is disposed on a downstream side of the intake space Si in a rotation direction of the fiber integration drum 12 (see arrow K1 in Fig. 1). The blowout space So is a (positive-pressure) space having a higher pressure than the exterior of the fiber integration drum 12, air being blown into the interior of the blowout space So through an opening (not shown). Air is blown out from the blowout space So to the space outside of the fiber integration drum 12 via the holes 12b formed in the bottom sections of the recesses 12a. Positions of the intake space Si and the blowout space So inside the fiber integration drum 12 do not change when the fiber integration drum 12 rotates.

The second opening 24 of the duct 20 faces a portion of the outer peripheral surface of the fiber integration drum 12 at a site where the intake space Si is formed in the interior of the fiber integration drum 12, the outer peripheral surface being positioned between the second opening 24 and the intake space Si. Because the fiber integration drum 12 rotates, the site on the outer peripheral surface of the fiber integration drum 12 facing the second opening 24 changes in association with rotation of the fiber integration drum 12. As the fiber integration drum 12 rotates and the recesses 12a formed in the outer peripheral surface of the fiber integration drum 12 move while facing the second opening 24 of the duct 20, the pulp-fiber-containing air supplied from the duct 20 flows into the recesses 12a. The air passes through the holes 12b formed in the recesses 12a and flows into the intake space Si, which has a lower pressure than a supply space Sa, but because the holes 12b are designed to dimensions such that the pulp fiber is not allowed to pass therethrough, the pulp fiber flowing into the recesses 12a does not flow into the intake space Si and instead is stacked in the recesses 12a.

The suction conveyor 30 is disposed adjacent to the blowout space So inside the fiber integration drum 12, the fiber integration drum 12 being interposed therebetween (see Fig. 1). The suction conveyor 30 is disposed on a downstream side of the intake space Si (see Fig. 1) in the rotation direction of the fiber integration drum 12 (see arrow K1). The suction conveyor 30 suctions air through a conveyor surface (transport surface), as indicated by arrows in Fig. 1. The suction conveyor 30 suctions the pulp fiber (absorbent bodies P) stacked in the recesses 12a in the outer peripheral surface of the fiber integration drum 12, releasing the absorbent bodies P from the recesses 12a, and transports the released absorbent bodies P. The absorbent bodies P transported by the suction conveyor 30 are transported to the step in the latter section and are used in disposable diapers, sanitary napkins, or the like.

### (2) Pulverization device

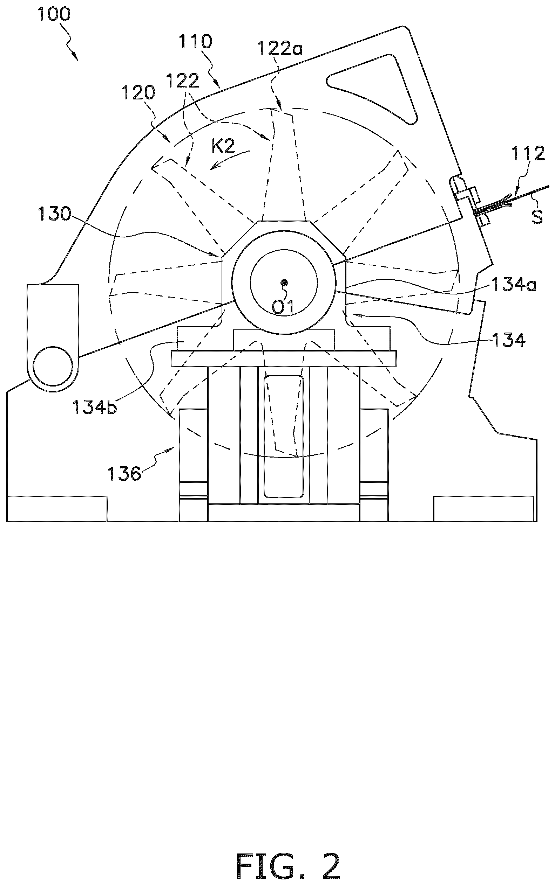

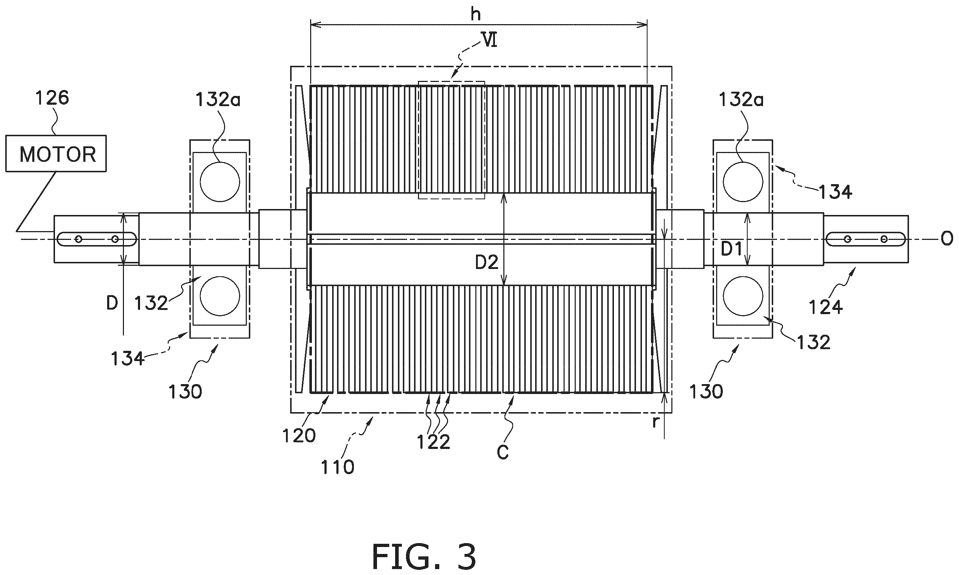

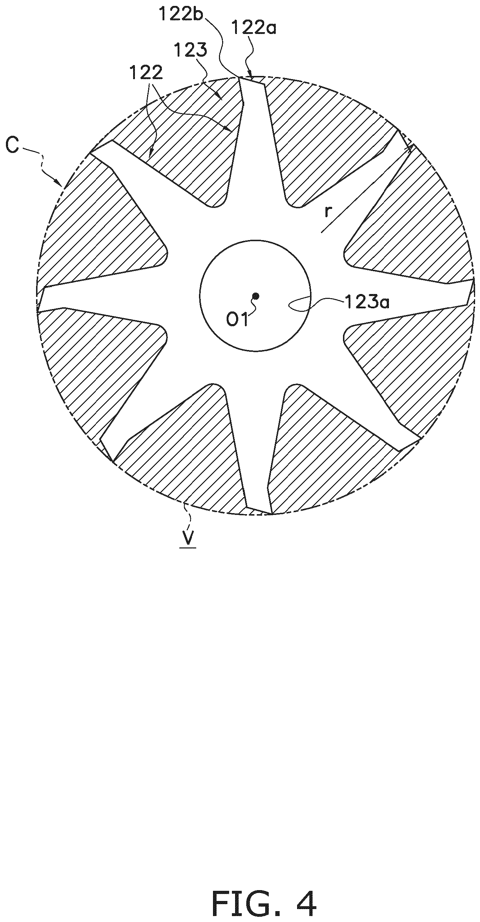

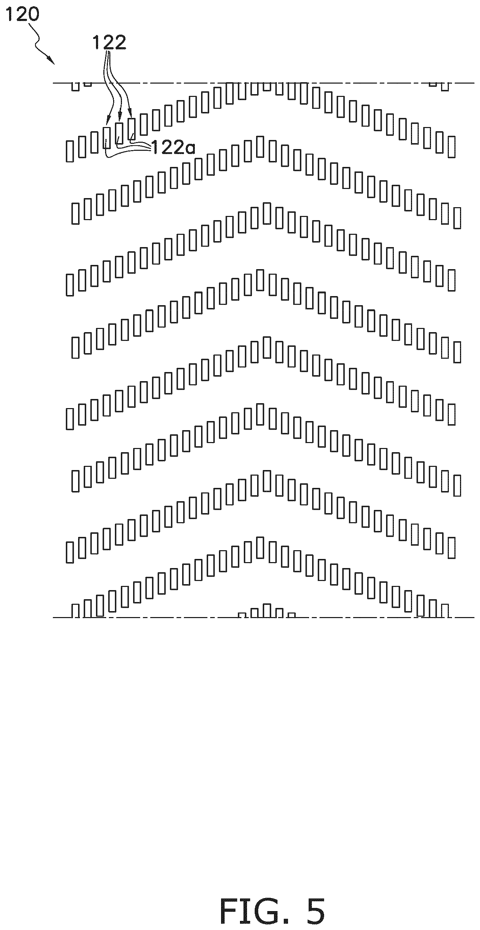

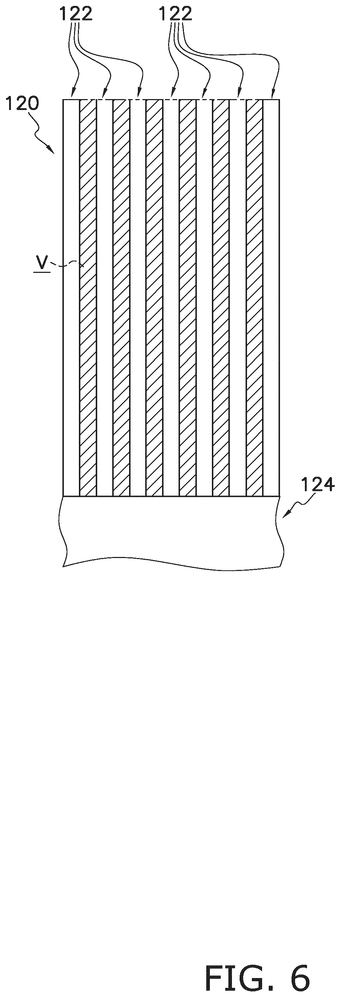

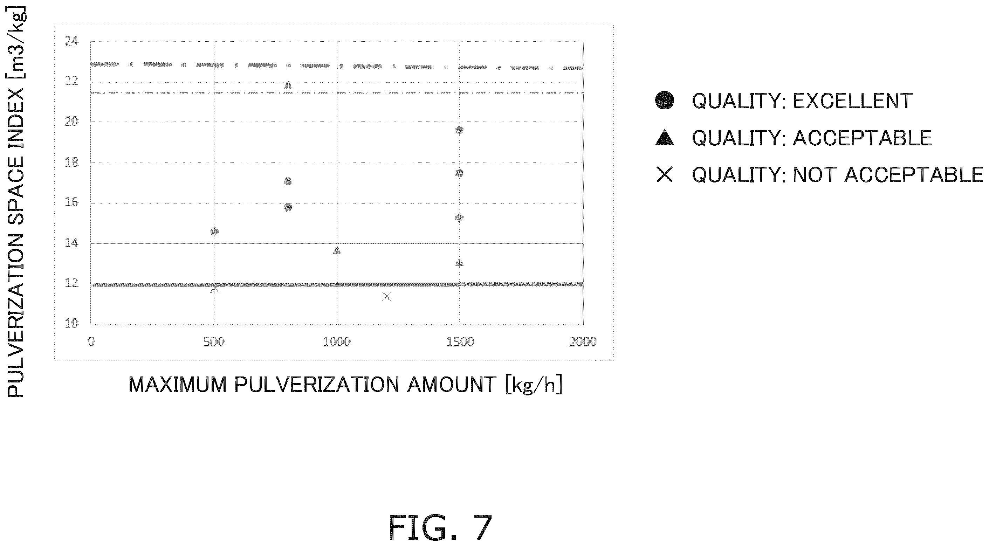

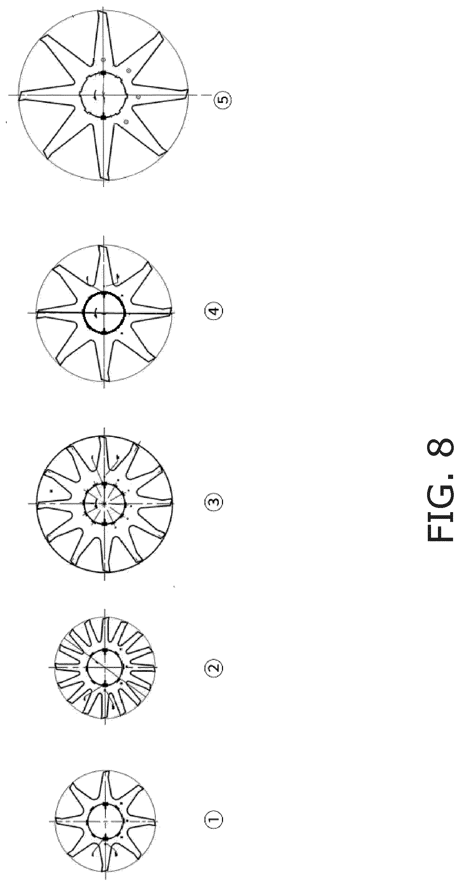

The specific configuration of the pulverization device 100 shall be described below with reference to Figs. 2 to 8. Fig. 2 is a schematic diagram in which the pulverization device 100 is viewed along an axial direction of a rotary shaft 124 (described further below) of the pulverization device 100. Fig. 3 is a schematic top view of a rotary body 120 and bearing devices 130 of the pulverization device 100, where a casing 110 and bearing boxes 134 (described further below) are illustrated using dashed and double-dotted lines. Specifically, Fig. 3 shows a view from a direction perpendicular to a direction in which the axial direction of the rotary shaft 124 extends. Fig. 4 is a schematic diagram in which rotary blades 122 (rotary blade plates 123) that are attached to the rotary shaft 124 are viewed along the axial direction of the rotary shaft 124. Fig. 5 is a diagram in which arcuate surfaces, where blade tips 122a of the rotary blades 122 (described further below) of the rotary body 120 are disposed, are illustrated in a planar manner, the diagram being for illustrating a state in which the blade tips 122a of the rotary blades 122 are disposed. Fig. 6 is a diagram in which a region including a quadrangle VI in Fig. 3 is partially enlarged, the diagram being for illustrating a void space. Fig. 7 is a diagram in which a maximum pulverization amount Amax and a pulverization space index I from when a pulp sheet S is pulverized using test equipment for the pulverization device (i.e., data shown in table 1) are plotted on a graph in which the maximum pulverization amount Amax is taken on a horizontal axis and the pulverization space index I is taken on a vertical axis, where symbols used for each plot indicate quality of resulting pulp fiber. Fig. 8 is a schematic diagram in which the rotary blades 122 (rotary blade plates 123) used in testing for obtaining the graph shown in Fig. 7 (i.e., data shown in table 1) are viewed along the axial direction of the rotary shaft 124.

The pulverization device 100 is mainly equipped with the casing 110, the rotary body 120, and the bearing devices 130 (see Fig. 2).

### (2-1) Rotary body

The rotary body 120 includes the rotary shaft 124, a plurality of the rotary blades 122, and a motor 126, as shown in Fig. 3.

The rotary shaft 124 is driven by the motor 126 to rotate (see arrow K2 indicating the rotation direction) about a rotational axis O (see Fig. 3) extending through a center O1 (see Fig. 2) of the rotary shaft 124.

The plurality of rotary blades 122 are attached to the rotary shaft 124. Despite not being limited in shape, the rotary blades 122 are formed so that the blade tips 122a (radially outer end sections of the rotary body 120) appear claw-shaped when the rotary body 120 is viewed along the axial direction of the rotary shaft 124.

The rotary blades 122 are, for example, attached to the rotary shaft 124 in the state described below, although this does not limit attachment state of the rotary blades 122 to the rotary shaft 124.

As shown in Fig. 4, the plurality of rotary blades 122 are disposed along a circumferential direction of the rotary body 120, and a plurality of rotary blade plates 123 individually having an attachment hole 123a formed therein are provided to a central section of the rotary body 120. The number of rotary blades 122 provided to the rotary blade plates 123 illustrated in Fig. 4 is merely presented as an example; the number of rotary blades 122 provided to the rotary blade plates 123 is to be determined as appropriate. The plurality of rotary blade plates 123 are attached to the rotary shaft 124 along the axial direction of the rotary shaft 124 so as to be set apart from one another by a prescribed distance. Spacers (not shown) may be used in order to position the rotary blade plates 123 in the axial direction of the rotary shaft 124. Specifically, the rotary blade plates 123 are attached to the rotary shaft 124 such that the rotary shaft 124 is inserted through the attachment holes 123a, and the rotary blade plates 123 are fixed to the rotary shaft 124 using a prescribed method. The rotary blade plates 123 are each attached to the rotary shaft 124 while an angle thereof with respect to adjacent rotary blade plates 123 is offset such that positions of the blade tips 122a of the rotary blades 122 are offset as shown in Fig. 5 in a circumferential direction of the rotary shaft 124. However, in Fig. 2, in order to avoid making the diagram more complicated, only the rotary blade plate 123 disposed closest to the front in the drawing is illustrated.

When the rotary body 120 (the rotary shaft 124 to which the rotary blades 122 are attached) is driven to rotate by the motor 126, the plurality of rotary blades 122 attached to the rotary shaft 124 also rotate together with the rotary shaft 124 and come into contact with the pulp sheet S supplied to the pulverization device 100, pulverizing (breaking down) the pulp sheet S.

### (2-2) Bearing devices

The bearing devices 130 pivotally support the rotary body 120 so as to allow rotation thereof. More specifically, the bearing devices 130 rotatably support the rotary shaft 124 of the rotary body 120. The pulverization device 100 has a pair of bearing devices 130, and the rotary blades 122 (rotary blade plates 123) of the rotary body 120 are disposed between the pair of bearing devices 130.

Each of the bearing devices 130 mainly includes ball bearings 132, the bearing boxes 134, and a base 136, as shown in Figs. 2 and 3.

The ball bearings 132 rotatably support the rotary shaft 124. The ball bearings 132 are preferably deep-groove ball bearings. Axial offset between the pair of ball bearings 132 is more permissible when deep-groove ball bearings are used than when other types of ball bearings are used.

Steel balls may be used as rolling elements of the ball bearings 132. However, when the rotary shaft 124 has a high rate of rotation set using a method described below (e.g., when the rotary shaft 124 is operated at a rate of rotation exceeding 3,500 rpm (4,000 rpm, etc.)), the rolling elements of the ball bearings 132 are preferably ceramic balls 132a. Additionally, in cases where a diameter of the rotary shaft 124 is increased as the rate of rotation of the rotary shaft 124 increases, a DN value (product of an inside diameter [mm] of the ball bearings 132 and the rate of rotation [rpm] of the rotary shaft 124) also increases. In cases where the DN value exceeds 300,000 (e.g., when the DN value is 450,000), the rolling elements of the ball bearings 132 are preferably ceramic balls 132a. Using the ball bearings 132 in which the ceramic balls 132 are used as the rolling elements makes it easier to extend service life of the bearings even when the rate of rotation of the rotary shaft 124 or the DN value is high.

The inventors also discovered that decreases in efficiency resulting from sliding in the ball bearings 132 are easier to suppress when the ceramic balls 132a are used as the rolling elements than when steel balls are used as the rolling elements, and the loss of motive power is comparatively low in the former case even when the rotary shaft is rotated at high speed.

The ball bearings 132 are preferably grease-sealed bearings. Using grease-sealed ball bearings 132 makes it possible to make configurations around the ball bearings 132 simpler than with cases where oil-lubricated (oil-supplied) bearings are employed. In cases where oil-lubricated bearings are used, there is a concern that a fire accident or the like could result if oil flows into the casing 110 (described further below) for any reason. However, there is no such risk in the pulverization device 100 in which grease-sealed ball bearings 132 lacking any oil supply structure are used.

The bearings are not necessarily required to be grease-sealed ball bearings. For example, oil-lubricated high-speed angular ball bearings or the like may be employed as the bearings. However, using grease-sealed ball bearings presents the advantages described above.

Either a contact form or a non-contact form may be employed for a hermetic (sealing) structure of the ball bearings 132. However, it is preferable to employ a non-contact form. Employing a non-contact form for the hermetic structure makes it possible to reduce generation of frictional heat during high-speed rotation.

The bearing boxes 134 are housings that accommodate the ball bearings 132 therein. The bearing boxes 134 are not attached to the casing 110 that accommodates the rotary blades 122 therein, but rather are supported from below by the base 136. The bearing boxes 134 are disposed set apart from the casing 110 in the axial direction of the rotary shaft 124 so that spaces are formed between the bearing boxes 134 and the casing 110. Specifically, the ball bearings 132 are disposed set apart from the casing 110 in the axial direction of the rotary shaft 124 so that spaces are formed between the ball bearings 132 and the casing 110. For example, the ball bearings 132 (more specifically the bearing boxes 134) and the casing 110 are preferably disposed set apart from one another by a distance of 1/6 to 2 times a radius r (described further below) (distance to an end section 122b of the rotary blade 122 disposed furthest away from the center O1 of the rotary shaft 124 in a radial direction of the rotary shaft 124). Due to this configuration, it is possible to suppress any adverse effects on performance of the ball bearings 132 due to heat within the casing 110 while suppressing any excessive increase in size of the pulverization device 100.

When viewed along the axial direction of the rotary shaft 124, the bearing box 134 preferably has a main body section 134a that accommodates the ball bearings 132, and leg sections 134b that project in a direction (i.e., left-right direction in Fig. 2) orthogonal to the axial direction of the rotary shaft 124. The leg sections 134b of the bearing box 134 are fixed to the base 136, the leg sections 134b supporting the ball bearings 132 in the direction (left-right direction in Fig. 2) orthogonal to the axial direction of the rotary shaft 124 in addition to an up-down direction.

### (2-3) Casing

The casing 110 is a housing that accommodates the rotary blades 122 (rotary blade plates 123) attached to the rotary shaft 124, as shown in Fig. 2. The casing 110 has a supply port 112 through which the pulp sheet S supplied by a sheet transport device (not shown) is supplied, and a discharge port 114 through which the pulp fiber produced due to the rotary blades 122 pulverizing the pulp sheet S is discharged, as shown in Fig. 1.

### (2-4) Setting of parameters for pulverization device

As a typical object to be addressed in the pulverization device 100, there is demand to realize high processing capacity in a compact device.

Moreover, in the pulverization device 100, there is also demand for the produced pulp fiber to meet quality standards. Specifically, for example, there is demand for the produced pulp fiber to not undergo any burning. Additionally, for example, there is demand for the amount of non-broken-down pulp fiber mixed into the produced pulp fiber to be at or below a given amount. Moreover, it is preferable for the produced pulp fiber to have a fixed fiber length (for the fiber to not be too finely pulverized). Conventionally, having the produced pulp fiber meet the quality standards has been prioritized, and pulverization devices have actually been designed to have considerable tolerances in terms of device size and processing capacity.

However, the inventors discovered that quantifying a condition under which both the problem of realizing high processing capacity in a compact device and the problem of having the produced pulp fiber meet the quality standards are satisfied makes it possible to suppress adverse events such as the pulp fiber burning or pulverization of the pulp sheet being inadequate and makes it possible to provide a pulverization device that is compact and has high processing capacity.

More specifically, the inventors discovered that defining the value of a pulverization space index I [m³/kg], and setting a manufacturing value (specifically a volume V [m³] of a void space within a virtual cylinder (described further below)) of the pulverization device 100, a maximum pulverization amount Amax [kg/h], and the rate of rotation [rpm] of the rotary shaft 124 so that the pulverization space index I satisfies a prescribed numerical condition, makes it possible to meet the quality standards for pulp fiber.

Definitions of the pulverization space index I [m³/kg], the volume V [m³] of the void space within the virtual cylinder C, and the maximum pulverization amount Amax [kg/h] shall be described first.

The pulverization space index I [m³/kg] is an amount defined using numerical expression (V [m³] × N [rpm] × 60 [min]) ÷ Amax [kg/h].

The virtual cylinder C appearing in the definition of the void space is an imaginary cylinder (see portion shown using bold dashed and dotted lines in Fig. 3) for which a radius r is a distance from the center O1 of the rotary shaft 124 to an end section 122b of the rotary blade 122 disposed furthest away from the center O1 of the rotary shaft 124 in the radial direction of the rotary shaft 124 (see Fig. 4) and for which a height h is a segment distance within which the plurality of rotary blades 122 are disposed in an axial direction of the rotary shaft 124 (see Fig. 3).

The volume V [m³] of the void space within the virtual cylinder C refers to a volume of a portion within the virtual cylinder C in which no member is present at all. Specifically, the volume V [m³] of the void space within the virtual cylinder C refers to a volume of a hollow section within the virtual cylinder C. for example, as shown in Fig. 4, when the rotary blade plates 123 are viewed along the axial direction of the rotary shaft 124, portions in which the rotary blades 122 are not present and which are indicated by hatching are included in the void space. Additionally, gaps between rotary blades 122 (i.e., gaps between adjacent rotary blade plates 123) that are adjacent to one another in the axial direction of the rotary shaft 124 are also included in the void space, the gaps being shown using hatching in Fig. 6. In cases where spacers are disposed between the adjacent rotary blade plates 123, the spaces in which the spacers are disposed are excluded from the void space.

The maximum pulverization amount Amax [kg/h] is a maximum design value for amount of pulverization (amount of processed pulp sheet S per hour) allowed in the pulverization device 100.

The inventors varied the shape of the rotary blade plates 123, the diameter (value that is two times the radius r of the virtual cylinder C) of the rotary blade plates 123, the rate of rotation of the rotary shaft 124, the maximum pulverization amount Amax [kg/h], and the volume V [m³] of the void space within the virtual cylinder C in the manner indicated in table 1 by using test equipment for the pulverization device 100, deriving a relationship between the pulverization space index I [m³/kg] and the quality of the pulp fiber (see table 1). The "shapes" category in table 1 refers to the shapes of the rotary blade plates 123, and the numeric characters in table 1 indicate which rotary blade plates 123 were used from among the rotary blade plates 123 shown in Fig. 8. The "pulverization space" category in table 1 indicates values defined using numerical expression V [m3] × N [rpm] × 60 [min].

**Table 1: Relationship between pulverization space index and pulp fiber quality**

| Shape | Diameter [mm] | Rate of rotation [rpm] | Maximum pulverization amount [kg/h] | Void space [m³] | Pulverization space [m³/h] | Pulverization space index [m³/kg] | Pulp fiber quality |
|---|---|---|---|---|---|---|---|
| 1 | 370 | 3500 | 500 | 0.0347 | 7592 | 14.6 | ○ |
| 2 | 370 | 3500 | 500 | 0.0280 | 5881 | 11.8 | × (Burnt) |
| 3 | 500 | 3500 | 800 | 0.0600 | 12607 | 15.8 | ○ |
| 4 | 500 | 3500 | 1200 | 0.0651 | 13668 | 11.4 | × (Much material not broken down) |
| | 500 | 3500 | 1000 | 0.0651 | 13668 | 13.7 | (Rather much material not broken down) |
| | 500 | 3500 | 800 | 0.0651 | 13668 | 17.1 | ○ |
| 5 | 625 | 3000 | 1500 | 0.1091 | 19636 | 13.1 | Δ (Rather much material not broken down) |
| | 625 | 3500 | 1500 | 0.1091 | 22909 | 15.3 | ○ |
| | 625 | 4000 | 1500 | 0.1091 | 26182 | 17.5 | ○ |
| | 625 | 4500 | 1500 | 0.1091 | 29455 | 19.6 | ○ |
| | 625 | 5000 | 1500 | 0.1091 | 32727 | 21.8 | Δ (Rather much material excessively broken down) |

Fig. 7 was obtained by plotting the maximum pulverization amount Amax and the pulverization space index I on a graph in which the maximum pulverization amount Amax is taken on a horizontal axis and the pulverization space index I is taken on a vertical axis, on the basis of the test results in table 1. Symbols used for plots in Fig. 7 correspond to those in the "pulp fiber quality" section in table 1 and indicate the quality of the resulting pulp fiber.

As a result of the above, the inventors discovered that having the pulverization space index I be equal to or greater than 12.0 m³/kg makes it possible to suppress the occurrence of adverse events such as the pulp fiber burning and pulverization of the pulp sheet S being markedly inadequate during pulverization (see table 1 and Fig. 7).

The inventors also discovered that having the pulverization space index I be equal to or greater than 14.0 m³/kg makes it possible to reduce the possibility that pulp fiber in a state of being a comparatively large clump that has not been adequately pulverized will be mixed into the produced pulp fiber (see table 1 and Fig. 7).

Furthermore, the inventors discovered that having the pulverization space index I be equal to or greater than 21.5 m³/kg, or even more preferably 20.0 m³/kg, makes it possible to reduce the possibility that excessively pulverized pulp fiber will be mixed into the produced pulp fiber (see table 1 and Fig. 7).

On the basis of these results, the inventors discovered that it is preferable to set the volume V [m³], the rate of rotation N [rpm], and the maximum pulverization amount [kg/h] in the pulverization device 100 in the manner described below.

First, the inventors discovered that from the standpoint of avoiding high-magnitude adverse events from the standpoint of pulp fiber quality, it is desirable for the volume V [m³], the rate of rotation N [rpm], and the maximum pulverization amount Amax [kg/h] to be set so that the pulverization space index I [m³/kg] is equal to or greater than 12.0.

Furthermore, the inventors discovered that in terms of reducing the possibility that pulp fiber in a state of being a comparatively large clump that has not been adequately pulverized will be mixed into the produced pulp fiber, it is preferable for the volume V [m³], the rate of rotation N [rpm], and the maximum pulverization amount Amax [kg/h] to be set so that the pulverization space index I [m³/kg] is equal to or greater than 14.0.

Additionally, the inventors discovered that from the standpoint of reducing the possibility that excessively pulverized pulp fiber will be mixed into the produced pulp fiber, it is preferable for the volume V [m³], the rate of rotation N [rpm], and the maximum pulverization amount Amax [kg/h] to be set so that the pulverization space index I [m³/kg] is equal to or less than 21.5. The inventors moreover discovered that from the standpoint of reducing the possibility that excessively pulverized pulp fiber will be mixed into the produced pulp fiber, it is even more preferable for the volume V [m³], the rate of rotation N [rpm], and the maximum pulverization amount Amax [kg/h] to be set so that the pulverization space index I [m³/kg] is equal to or less than 20.0. The inventors also discovered that because the amount of excessively pulverized pulp fiber increases if the pulverization space index I [m³/kg] rises too high, it is preferable for the volume V [m³], the rate of rotation N [rpm], and the maximum pulverization amount Amax [kg/h] to be set so that the pulverization space index I [m³/kg] does not exceed 23.0.

Using the pulverization space index I as described above makes it possible to numerically ascertain the manner in which to set the volume V [m³], the rate of rotation N [rpm], and the maximum pulverization amount Amax [kg/h]. Therefore, in the pulverization device 100 according to the present embodiment, it is possible to suppress adverse events such as the pulp fiber burning or pulverization of the pulp sheet being inadequate and to realize a pulverization device that is compact and has high processing capacity.

### (3) Features

(3-1)
The pulverization device 100 according to the embodiment described above pulverizes the pulp sheet S and produces the pulp fiber. The pulverization device 100 includes the rotary shaft 124, the plurality of rotary blades 122, the motor 126, and the casing 110. The plurality of rotary blades 122 are attached to the rotary shaft 124, the rotary blades 122 rotating together with the rotary shaft 124 and pulverizing the pulp sheet S. The motor 126 causes the rotary shaft 124 to rotate. The casing 110 has the supply port 112 through which the pulp sheet S is supplied, and the discharge port 114 through which the pulp fiber is discharged. The casing 110 accommodates the rotary blades 122 attached to the rotary shaft 124. The pulverization space index I [m³/kg] is defined using numerical expression (V [m³] × N [rpm] × 60 [min]) ÷ Amax [kg/h], where V [m³] is the volume of the void space within the virtual cylinder, N [rpm] is the rate of rotation of the rotary shaft 124, and Amax [kg/h] is the maximum amount of material pulverized by the pulverization device. The virtual cylinder C is an imaginary cylinder for which the radius r is the distance from the center O1 of the rotary shaft 124 to the end section 122b of the rotary blade 122 disposed furthest away from the center O1 of the rotary shaft 124 in the radial direction of the rotary shaft 124 and for which the height h is the segment distance within which the plurality of rotary blades 122 are disposed in an axial direction of the rotary shaft 124. The volume V [m³], the rate of rotation N [rpm], and the maximum pulverization amount Amax [kg/h] in the definition above are set so that the pulverization space index I [m³/kg] is equal to or greater than 12.0.

The inventors discovered that having the pulverization space index I be equal to or greater than 12.0 m³/kg makes it possible to suppress the occurrence of adverse events such as the pulp fiber burning and pulverization of the pulp sheet S being markedly inadequate during pulverization (see table 1 and Fig. 7). Because the volume V [m³], the rate of rotation N [rpm], and the maximum pulverization amount Amax [kg/h] are set so that the aforementioned condition is satisfied in the pulverization device 100, it is possible to suppress the occurrence of adverse events such as the pulp fiber burning and pulverization of the pulp sheet S being markedly inadequate even without excessive safety tolerances.

The volume V [m³], the rate of rotation N [rpm], and the maximum pulverization amount [kg/h] in the pulverization device 100 are more preferably set so that the pulverization space index I [m³/kg] is equal to or greater than 14.0.

The inventors discovered that having the pulverization space index I be equal to or greater than 14.0 m³/kg makes it possible to reduce the possibility that pulp fiber in a state of being a comparatively large clump that has not been adequately pulverized will be mixed into the produced pulp fiber (see table 1 and Fig. 7). Due to the volume V [m³], the rate of rotation N [rpm], and the maximum pulverization amount Amax [kg/h] being set so that the aforementioned condition is satisfied in the pulverization device 100, pulp fiber in a state of being a comparatively large clump that has not been adequately pulverized is not readily mixed into the produced pulp fiber even without excessive safety tolerances.

(3-2)
The volume V [m³], the rate of rotation N [rpm], and the maximum pulverization amount [kg/h] in the pulverization device 100 are preferably set so that the pulverization space index I [m³/kg] is equal to or less than 23.0.

Due to the aforementioned condition being satisfied, it is possible to suppress the occurrence of adverse events such as the pulp sheet S being excessively pulverized and to produce high-quality pulp fiber using the pulverization device 100.

The volume V [m³], the rate of rotation N [rpm], and the maximum pulverization amount [kg/h] in the pulverization device 100 are more preferably set so that the pulverization space index I [m³/kg] is equal to or less than 21.5.

The inventors discovered that having the pulverization space index I [m³/kg] be equal to or greater than 21.5 makes it possible to reduce the possibility that excessively pulverized pulp fiber will be mixed into the produced pulp fiber (see table 1 and Fig. 7). Due to the volume V [m³], the rate of rotation N [rpm], and the maximum pulverization amount Amax [kg/h] being set so that the aforementioned condition is satisfied in the pulverization device 100, excessively pulverized pulp fiber is not readily mixed into the produced pulp fiber even without excessive safety tolerances.

(3-3)
The pulverization device 100 according to the embodiment described above pulverizes the pulp sheet S and produces the pulp fiber. The pulverization device 100 includes the rotary shaft 124, the plurality of rotary blades 122, the motor 126, the casing 110, and the ball bearings 132. The plurality of rotary blades 122 are attached to the rotary shaft 124, the rotary blades 122 rotating together with the rotary shaft 124 and pulverizing the pulp sheet S. The motor 126 causes the rotary shaft 124 to rotate. The casing 110 has the supply port 112 through which the pulp sheet S is supplied, and the discharge port 114 through which the pulp fiber is discharged. The casing 110 accommodates the rotary blades 122 attached to the rotary shaft 124. The rolling elements of the ball bearings 132 are the ceramic balls 132a.

Conventionally, steel balls are used as rolling elements of bearings that pivotally support a rotating shaft in pulverization devices. However, if the rotary shaft has an excessively high rate of rotation in such pulverization devices, then the bearings will be more readily damaged due to frictional heat, and adverse events such as efficiency decreasing could occur.

However, if the ceramic balls 132a are used for the rolling elements of the ball bearings 132, then the rotary shaft 124 will be able to rotate at a greater rate of rotation than with conventional pulverization devices because ceramic is resistant to heat, and it will be easy to suppress damage to the ball bearings 132 caused by frictional heat even when the pulverization device is operated under high load. Additionally, the inventors discovered that using the ceramic balls 132a for the rolling elements of the ball bearings 132 also makes it possible to suppress a decrease in efficiency associated with sliding in the ball bearings 132.

If the ceramic balls 132a are used for the rolling elements of the ball bearings 132, then the service life of the bearings will not readily decrease even when the rotary shaft 124 is operated at a high rate of rotation such that the pulverization space index I [m³/kg] in the pulverization device 100 satisfies the numeric values described above and the DN value (dimensionless amount that is the product of a bearing inside diameter [mm] and the rate of rotation [rpm] of the rotary shaft 124) is high.

(3-4)
In the pulverization device 100 according to the embodiment described above, the ball bearings 132 are grease-sealed bearings.

Because no oil-lubricated ball bearings are used in the pulverization device 100, structures around the ball bearings 132 can be simplified.

In cases where oil-lubricated bearings are used, there is a concern that a fire accident or the like could result if oil flows into the casing 110 for any reason. However, there is no such risk in the pulverization device 100 in which grease-sealed ball bearings 132 lacking any oil supply structure are used.

(3-5)
In the pulverization device 100 according to the embodiment described above, the hermetic (sealing) structure of the ball bearings 132 is preferably in a non-contact form.

Employing a non-contact-form hermetic structure makes it possible to reduce generation of frictional heat during high-speed rotation associated with the presence of the hermetic structure.

(3-6)
In the pulverization device 100 according to the embodiment described above, the ball bearings 132 are disposed set apart from the casing 110 so that spaces are formed between the ball bearings 132 and the casing 110. More specifically, the bearing boxes 134 that accommodate the ball bearings 132 are disposed set apart from the casing 110 so that spaces are formed between the ball bearings 132 and the casing 110.

In the pulverization device 110, a temperature within the casing 110 readily rises. Therefore, in cases where the ball bearings 132 (the bearing boxes 134 that accommodate the ball bearings 132) are in contact with the casing 110, there is a concern that heat within the casing 110 could adversely affect performance of the ball bearings 132. However, due to the ball bearings 132 being disposed set apart from the casing 110, the ball bearings 132 are not readily affected by heat within the casing 110.

### (4) Modifications

### (4-1) Modification A

Shapes of the leg sections 134b and the base 136 of the bearing box 134 are not limited to the shapes illustrated in Fig. 2.

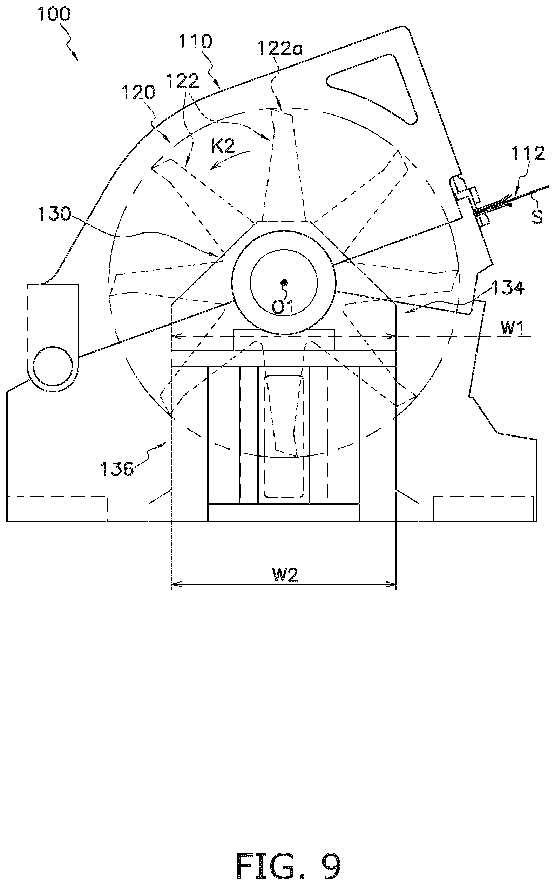

For example, widths of the leg sections 134b and the base 136 of the bearing box 134 in the left-right direction when viewed along the axial direction of the rotary shaft 124 are preferably comparatively large, as shown in Fig. 9.

For example, a maximum width W1 of the leg sections 134b of the bearing box 134 is preferably at least 3 times but less than 6 times a diameter D1 (see Fig. 3) of a portion of the rotary shaft 124 supported by the ball bearings 132.

Additionally, for example, an average width W2 of the base 136 of the bearing box 134 is preferably at least 3 times but less than 6 times the diameter D1 of the portion of the rotary shaft 124 supported by the ball bearings 132.

Due to this configuration, it is easy to suppress vibration even when the rotary shaft 124 rotates at high speed, and it is possible to suppress any damage to the ball bearings 132 and any decrease in efficiency of the pulverization device 100.

### (4-2) Modification B

In the example illustrated in Fig. 3, a diameter D2 of a portion of the rotary shaft 124 at which the rotary blades 122 are attached and which is accommodated within the casing 110 is set greater than the diameter D1 of the portion of the rotary shaft 124 supported by the ball bearings 132 (i.e., diameter D1 < diameter D2). However, the present invention is not limited to the shape illustrated in Fig. 3, and it is also permissible to employ a shape satisfying the relationship diameter D1 ≥ diameter D2. However, a shape in which diameter D1 < diameter D2 is preferred because this shape makes it possible to prevent the occurrence of resonance and a reduction in flexing.

### <Notes>

Finally, a technical concept that can be ascertained from the embodiments described above shall be appended below.

A pulverization device according to a first aspect of the present invention pulverizes a pulp sheet and produces pulp fiber. The pulverization device comprises a rotary shaft, a plurality of rotary blades, a motor, and a casing. The plurality of rotary blades are attached to the rotary shaft, the rotary blades rotating together with the rotary shaft and pulverizing the pulp sheet. The motor causes the rotary shaft to rotate. The casing has a supply port through which the pulp sheet is supplied and a discharge port through which the pulp fiber is discharged. The casing accommodates the rotary blades attached to the rotary shaft. A pulverization space index I [m³/kg] is defined using numerical expression (V [m³] × N [rpm] × 60 [min]) ÷ Amax [kg/h], where V [m³] is a volume of a void space within a virtual cylinder, N [rpm] is a rate of rotation of the rotary shaft, and Amax [kg/h] is a maximum amount of material pulverized by the pulverization device. The virtual cylinder is an imaginary cylinder for which a radius is a distance from a center of the rotary shaft to an end section of the rotary blade disposed furthest away from the center of the rotary shaft in a radial direction of the rotary shaft and for which a height is a segment distance within which the plurality of rotary blades are disposed in an axial direction of the rotary shaft. The volume V [m³], the rate of rotation N [rpm], and the maximum pulverization amount Amax [kg/h] in the definition above are set so that the pulverization space index I [m³/kg] is equal to or greater than 12.0.

The inventors discovered that having the pulverization space index I be equal to or greater than 12.0 m³/kg makes it possible to suppress the occurrence of adverse events such as the pulp fiber burning and pulverization of the pulp sheet being markedly inadequate during pulverization. Because the volume V [m³], the rate of rotation N [rpm], and the maximum pulverization amount Amax [kg/h] are set so that the aforementioned condition is satisfied in the pulverization device according to the first aspect, it is possible to suppress the occurrence of adverse events such as the pulp fiber burning and pulverization of the pulp sheet being markedly inadequate even without excessive safety tolerances.

A pulverization device according to a second aspect of the present invention is the pulverization device according to the first aspect, wherein the volume V [m³], the rate of rotation N [rpm], and the maximum pulverization amount [kg/h] are set so that the pulverization space index I [m³/kg] is equal to or greater than 14.0.

The inventors discovered that having the pulverization space index I be equal to or greater than 14.0 m³/kg makes it possible to reduce the possibility that pulp fiber in a state of being a comparatively large clump that has not been adequately pulverized will be mixed into the produced pulp fiber. Because the volume V [m³], the rate of rotation N [rpm], and the maximum pulverization amount Amax [kg/h] are set so that the aforementioned condition is satisfied in the pulverization device according to the second aspect, pulp fiber in a state of being a comparatively large clump that has not been adequately pulverized is not readily mixed into the produced pulp fiber even without excessive safety tolerances.

A pulverization device according to a third aspect of the present invention is the pulverization device according to the first or second aspect, wherein the volume V [m³], the rate of rotation N [rpm], and the maximum pulverization amount [kg/h] are set so that the pulverization space index I [m³/kg] is equal to or less than 23.0.

It is possible to suppress the occurrence of adverse events such as the pulp sheet being excessively pulverized and to produce high-quality pulp fiber using the pulverization device according to the third aspect.

A pulverization device according to a fourth aspect of the present invention is the pulverization device according to the third aspect, wherein the volume V [m³], the rate of rotation N [rpm], and the maximum pulverization amount [kg/h] are set so that the pulverization space index I [m³/kg] is equal to or less than 21.5.

The inventors discovered that having the pulverization space index I [m³/kg] be equal to or greater than 21.5 makes it possible to reduce the possibility that excessively pulverized pulp fiber will be mixed into the produced pulp fiber. Because the volume V [m³], the rate of rotation N [rpm], and the maximum pulverization amount Amax [kg/h] are set so that the aforementioned condition is satisfied in the pulverization device according to the fourth aspect, excessively pulverized pulp fiber is not readily mixed into the produced pulp fiber even without excessive safety tolerances, and it is possible to provide higher-quality pulp fiber.

A pulverization device according to a fifth aspect of the present invention is the pulverization device according to any one of the first to fourth aspects, wherein the pulverization device further include a ball bearing that rotatably supports the rotary shaft. A rolling element of the ball bearing is a ceramic ball.

In the pulverization device according to the fifth aspect, service life of the bearing will not readily decrease and highly efficient operation will easily be realized even when the rotary shaft is rotated at a high rate of rotation and the pulverization device is operated under a high load.

A pulverization device according to a sixth aspect of the present invention is the pulverization device according to the fifth aspect, wherein the ball bearings are grease-sealed bearings.

Because no oil-lubricated ball bearings are used in the pulverization device according to the sixth aspect, structures around the ball bearings can be simplified.

In cases where oil-lubricated bearings are used, there is a concern that a fire accident or the like could result if oil flows into the casing for any reason. However, there is no such risk in the pulverization device in which grease-sealed ball bearings lacking any oil supply structure are used.

A pulverization device according to a seventh aspect of the present invention is the pulverization device according to the fifth or sixth aspect, wherein the ball bearings are disposed set apart from the casing so that spaces are formed between the ball bearings and the casing.

In the pulverization device, a temperature within the casing readily rises. Therefore, in cases where the ball bearings are in contact with the casing, there is a concern that heat within the casing could adversely affect performance of the ball bearings. However, due to the ball bearings being disposed set apart from the casing in the pulverization device according to the seventh aspect, the ball bearings are not readily affected by heat within the casing.

A pulverization device according to an eighth aspect of the present invention is the pulverization device according to any one of the fifth to seventh aspects, wherein a hermetic (sealing) structure of the ball bearings is preferably in a non-contact form.

Employing a non-contact-form hermetic structure in the pulverization device according to the eighth aspect makes it possible to reduce generation of frictional heat during high-speed rotation.

### REFERENCE SIGNS LIST

- 100: Pulverization device
- 110: Casing
- 112: Supply port
- 114: Discharge port
- 124: Rotary shaft
- 122: Rotary blade
- 122b: End section
- 126: Motor
- 132: Ball bearing
- 132a: Ceramic ball
- 134: Bearing box
- Amax: Maximum pulverization amount
- C: Virtual cylinder
- H: Height
- I: Pulverization space index
- N: Rate of rotation
- r: Radius
- S: Pulp sheet
- V: Volume of void space

## Claims

1. A pulverization device (100) that pulverizes a pulp sheet and produces pulp fiber, the pulverization device comprising:
a rotary shaft (124);
a plurality of rotary blades (122) attached to the rotary shaft, the rotary blades being configured to rotate together with the rotary shaft and pulverize the pulp sheet;
a motor (126) configured to cause the rotary shaft to rotate; and
a casing (110) having a supply port (112) through which the pulp sheet is configured to be supplied and a discharge port (114) through which the pulp fiber is configured to be discharged, the casing accommodating the rotary blades attached to the rotary shaft, **characterized in that**
a pulverization space index I [m³/kg] is defined using numerical expression (V [m³] × N [rpm] × 60 [min]) ÷ Amax [kg/h],
where V [m³] is a volume of a void space within a virtual cylinder, for which a radius is a distance from a center of the rotary shaft to an end section (112b) of the rotary blade disposed furthest away from the center of the rotary shaft in a radial direction of the rotary shaft and for which a height is a segment distance within which the plurality of rotary blades are disposed in an axial direction of the rotary shaft, when the virtual cylinder is virtualized,
N [rpm] is a rate of rotation of the rotary shaft, and
Amax [kg/h] is a maximum amount of material pulverized by the pulverization device, and
the volume V [m³], the rate of rotation N [rpm], and the maximum pulverization amount Amax [kg/h] being set so that the pulverization space index I [m³/kg] is equal to or greater than 12.0.

2. The pulverization device according to claim 1, wherein
the volume V [m³], the rate of rotation N [rpm], and the maximum pulverization amount [kg/h] are set so that the pulverization space index I [m³/kg] is equal to or greater than 14.0.

3. The pulverization device according to claim 1 or 2, wherein
the volume V [m³], the rate of rotation N [rpm], and the maximum pulverization amount [kg/h] are set so that the pulverization space index I [m³/kg] is equal to or less than 23.0.

4. The pulverization device according to claim 3, wherein
the volume V [m³], the rate of rotation N [rpm], and the maximum pulverization amount [kg/h] are set so that the pulverization space index I [m³/kg] is equal to or less than 21.5.

5. The pulverization device according to any of claims 1 to 4, further comprising
a ball bearing (132) configured to rotatably support the rotary shaft, and
a rolling element of the ball bearings is a ceramic ball (132a).

6. The pulverization device according to claim 5, wherein
the ball bearings are grease-sealed bearings.

7. The pulverization device according to claim 5 or 6, wherein
the ball bearings are disposed set apart from the casing so that spaces are formed between the ball bearings and the casing.

8. The pulverization device according to any of claims 5 to 7, wherein
a hermetic structure of the ball bearings is in a non-contact form.

## Patentansprüche

1. Eine Zerkleinerungsvorrichtung (100), die eine Zellstoffbahn zerkleinert und Zellstofffasern erzeugt, wobei die Zerkleinerungsvorrichtung umfasst:
eine Drehwelle (124);
eine Vielzahl von an der Drehwelle befestigten Rotationsklingen (122), wobei die Rotationsklingen so ausgebildet sind, dass sie sich zusammen mit der Drehwelle drehen und die Zellstoffbahn zerkleinern;
einen Motor (126), der so ausgelegt ist, dass er die Drehwelle in Drehung versetzt; und
ein Gehäuse (110) mit einer Zuführöffnung (112), durch die die Zellstoffbahn zugeführt wird, und einer Auslassöffnung (114), durch die die Zellstofffasern ausgetragen werden, wobei das Gehäuse die an der Drehwelle befestigten Rotationsschaufeln aufnimmt, **dadurch gekennzeichnet, dass**
Ein Zerkleinerungsraumindex I [m³/kg] wird anhand der folgenden Formel definiert: (V [m³] × N [U/min] × 60 [min]) ÷ Amax [kg/h],
wobei V [m³] das Volumen eines Hohlraums innerhalb eines virtuellen Zylinders ist, dessen Radius der Abstand vom Mittelpunkt der Drehwelle zu einem Endabschnitt (112b) des Rotationsflügels, der in radialer Richtung der Rotationswelle am weitesten von deren Mittelpunkt entfernt angeordnet ist, und dessen Höhe der Streckenabschnitt in axialer Richtung der Rotationswelle ist, innerhalb dessen die mehreren Rotationsflügel angeordnet sind, wenn der virtuelle Zylinder virtualisiert wird,
N [U/min] ist die Drehzahl der Drehwelle, und
Amax [kg/h] ist die maximale Materialmenge, die von der Zerkleinerungsvorrichtung zerkleinert wird, und
wobei das Volumen V [m³], die Drehzahl N [U/min] und die maximale Zerkleinerungsmenge Amax [kg/h] so eingestellt werden, dass der Zerkleinerungsraumindex I [m³/kg] mindestens 12,0 beträgt.

2. Die Zerkleinerungsvorrichtung nach Anspruch 1, wobei
Das Volumen V [m³], die Drehzahl N [U/min] und die maximale Zerkleinerungsmenge [kg/h] werden so eingestellt, dass der Zerkleinerungsraumindex I [m³/kg] mindestens 14,0 beträgt.

3. Die Zerkleinerungsvorrichtung nach Anspruch 1 oder 2, wobei
Das Volumen V [m³], die Drehzahl N [U/min] und die maximale Zerkleinerungsmenge [kg/h] werden so eingestellt, dass der Zerkleinerungsraumindex I [m³/kg] höchstens 23,0 beträgt.

4. Die Zerkleinerungsvorrichtung nach Anspruch 3, wobei
Das Volumen V [m³], die Drehzahl N [U/min] und die maximale Zerkleinerungsmenge [kg/h] werden so eingestellt, dass der Zerkleinerungsraumindex I [m³/kg] höchstens 21,5 beträgt.

5. Zerkleinerungsvorrichtung nach einem der Ansprüche 1 bis 4, die ferner umfasst:
ein Kugellager (132), das so ausgebildet ist, dass es die Drehwelle drehbar lagert, und
wobei ein Wälzkörper des Kugellagers eine Keramikkugel (132a) ist.

6. Die Zerkleinerungsvorrichtung nach Anspruch 5, wobei
die Kugellager fettgedichtete Lager sind.

7. Zerkleinerungsvorrichtung nach Anspruch 5 oder 6, wobei
die Kugellager so vom Gehäuse beabstandet angeordnet sind, dass zwischen den Kugellagern und dem Gehäuse Zwischenräume gebildet werden.

8. Zerkleinerungsvorrichtung nach einem der Ansprüche 5 bis 7, wobei
die hermetische Abdichtung der Kugellager berührungslos ausgebildet ist.

## Revendications

1. Dispositif de pulvérisation (100) destiné à pulvériser une feuille de pâte à papier et à produire des fibres de pâte à papier, ledit dispositif de pulvérisation comprenant :
un arbre rotatif (124) ;
une pluralité de lames rotatives (122) fixées à l'arbre rotatif, lesdites lames rotatives étant conçues pour tourner conjointement avec l'arbre rotatif et pulvériser la feuille de pâte ;
un moteur (126) conçu pour entraîner la rotation de l'arbre rotatif ; et
un carter (110) comportant un orifice d'alimentation (112) par lequel la feuille de pâte est destinée à être amenée et un orifice d'évacuation (114) par lequel la fibre de pâte est destinée à être évacuée, le carter logeant les lames rotatives fixées à l'arbre rotatif, **caractérisé en ce que**
un indice d'espace de pulvérisation I [m³/kg] est défini à l'aide de l'expression numérique (V [m³] × N [tr/min] × 60 [min]) ÷ Amax [kg/h],
où V [m³] représente le volume d'un espace vide à l'intérieur d'un cylindre virtuel, dont le rayon correspond à la distance entre le centre de l'arbre rotatif et une extrémité (112b) de la pale rotative la plus éloignée du centre de l'arbre rotatif dans la direction radiale de celui-ci, et dont la hauteur correspond à la distance sur laquelle la pluralité de pales rotatives est disposée dans la direction axiale de l'arbre rotatif, lorsque le cylindre virtuel est virtualisé,
N [tr/min] correspond à la vitesse de rotation de l'arbre rotatif, et
Amax [kg/h] correspond à la quantité maximale de matière pulvérisée par le dispositif de pulvérisation, et
le volume V [m³], la vitesse de rotation N [tr/min] et le débit maximal de pulvérisation Amax [kg/h] étant réglés de manière à ce que l'indice d'espace de pulvérisation I [m³/kg] soit égal ou supérieur à 12,0.

2. Dispositif de pulvérisation selon la revendication 1, **caractérisé en ce que**
Le volume V [m³], la vitesse de rotation N [tr/min] et le débit maximal de pulvérisation [kg/h] sont réglés de manière à ce que l'indice d'espace de pulvérisation I [m³/kg] soit égal ou supérieur à 14,0.

3. Dispositif de pulvérisation selon la revendication 1 ou 2, **caractérisé en ce que**
Le volume V [m³], la vitesse de rotation N [tr/min] et le débit maximal de pulvérisation [kg/h] sont réglés de manière à ce que l'indice de volume de pulvérisation I [m³/kg] soit inférieur ou égal à 23,0.

4. Dispositif de pulvérisation selon la revendication 3, **caractérisé en ce que**
Le volume V [m³], la vitesse de rotation N [tr/min] et le débit maximal de pulvérisation [kg/h] sont réglés de manière à ce que l'indice d'espace de pulvérisation I [m³/kg] soit inférieur ou égal à 21,5.

5. Dispositif de pulvérisation selon l'une quelconque des revendications 1 à 4, comprenant en outre
un roulement à billes (132) conçu pour supporter de manière rotative l'arbre rotatif, et
L'un des éléments roulants des roulements à billes est une bille en céramique (132a).

6. Dispositif de pulvérisation selon la revendication 5, **caractérisé en ce que**
Les roulements à billes sont des roulements étanches à la graisse.

7. Dispositif de pulvérisation selon la revendication 5 ou 6, **caractérisé en ce que**
Les roulements à billes sont disposés à distance du boîtier, de manière à former des espaces entre les roulements à billes et le boîtier.

8. Dispositif de pulvérisation selon l'une quelconque des revendications 5 à 7, **caractérisé en ce que**
La structure hermétique des roulements à billes est de type sans contact.
